# EUROPEAN PATENT SPECIFICATION

(11) **EP 2 400 967 B1**
(45) Date of publication and mention of the grant of the patent: **14.08.2019**
(21) Application number: 10707436.1
(22) Date of filing: 26.02.2010
(51) Int. Cl.: A61K 31/473, A61K 31/4741, A61P 25/14, A61P 25/16

(54) **TREATMENT OF DYSKINESIA RELATED DISORDERS**
BEHANDLUNG VON DYSKINESIEBEDINGTEN STÖRUNGEN
TRAITEMENT DE TROUBLES LIÉS À LA DYSKINÉSIE

(30) Priority: 27.02.2009 DK 200900281; 27.02.2009 US 155943 P; 27.02.2009 DK 200900273; 27.02.2009 US 155953 P; 27.02.2009 DK 200900280; 27.02.2009 US 155966 P
(43) Date of publication of application: 04.01.2012
(73) Proprietor: H. Lundbeck A/S, 2500 Valby (DK)
(72) Inventor: WIKSTRÖM, Håkan, S-45070 Hamburgsund (SE); JØRGENSEN, Morten, DK-2880 Bagsværd (DK); MØRK, Niels, DK-2830 Virum (DK); LARSEN, Jennifer, DK-4000 Roskilde (DK); TORUP, Lars, DK-3500 Værløse (DK); BANG-ANDERSEN, Benny, DK-2300 Copenhagen S (DK)
(74) Representative: H. Lundbeck A/S
(86) International application number: PCT/DK2010/050051
(87) International publication number: WO 2010/097092

(56) References cited:
- WO-A1-01/78713
- WO-A1-2009/026934
- STOCCHI FABRIZIO ET AL: "Continuous dopaminergic stimulation in early and advanced Parkinson's disease." NEUROLOGY, vol. 62, no. 1 Supplement 1, 13 January 2004 (2004-01-13), pages S56-S63, XP002576738 ISSN: 0028-3878 cited in the application
- STOCCHI F ET AL: "Continuous dopaminergic stimulation in early and advanced Parkinson's disease", NEUROLOGY, LIPPINCOTT WILLIAMS & WILKINS, PHILADELPHIA, US, vol. 62, no. 1 Supplement 1, 13 January 2004 (2004-01-13), pages S56-S63, XP002576738, ISSN: 0028-3878
- Hilary L. Tyne ET AL: "A 10 year retrospective audit of long-term apomorphine use in Parkinson?s disease", JOURNAL OF NEUROLOGY - ZEITSCHRIFT FUER NEUROLOGIE, vol. 251, no. 11, 1 November 2004 (2004-11-01), pages 1370-1374, XP055311973, DE ISSN: 0340-5354, DOI: 10.1007/s00415-004-0547-4

## Description

### FIELD OF THE INVENTION

Aspects of the subject invention relate to methods of treating Parkinson's disease while maintaining a low dyskinesia induction profile and to methods of reversing dyskinesias comprising administering therapeutically effective amount of a compound disclosed herein. The present invention further relates to uses and pharmaceutical compositions of said compounds in the manufacture of medicaments in treating the same or other movement disorders such as Huntington's chorea.

### BACKGROUND ART

The use of dopamine-replacing agents in the symptomatic treatment of Parkinson's disease (PD) has undoubtedly been successful in increasing the quality of life of patients. L-DOPA, which has been used for many years and remains the gold standard for treatment of PD, alleviates motor symptoms of PD characterized by the slowness of movement (bradykinesia), rigidity and/or tremor. It is understood that L-DOPA acts as a prodrug which is bio-metabolized into dopamine (DA). DA in turn activates dopamine receptors in the brain which fall into two classes: D1 and D2 receptors. D1 receptors can be divided into D₁ and D₅ receptors while D2 receptors can be divided into D₂, D₃, and D₄ receptors. However, dopamine-replacement therapy does have limitations, especially following long-term treatment. The duration period response to a dose of L-DOPA becomes progressively shorter over the years, and periods in which the patient responds to the drug become complicated by the appearance of a range of side-effects.

The side-effects may manifest as dyskinesias, which can be seen either when the patient is undergoing dopamine replacement therapy or even when the patient is off therapy. Dyskinesias are abnormal involuntary movement disorders. The abnormal movements may manifest as chorea (involuntary, rapid, irregular, jerky movements that may affect the face, arms, legs, or trunk), ballism (involuntary movements similar to chorea but of a more violent and forceful nature), dystonia (sustained muscle contractions, usually producing twisting and repetitive movements or abnormal postures or positions) and/or athetosis (repetitive involuntary, slow, sinuous, writhing movements, which are especially severe in the hands).

PD afflicted patients may cycle between "on" periods which are complicated by dyskinesia and "off" periods in which they are severely parkinsonian. As a consequence they may experience profound disability despite the fact that L-DOPA remains an effective anti-Parkinson agent throughout the course of the disease (Obeso, et al. Neurology 2000, 55, S13-23). Dopamine agonists such as bromocriptine, lisuride, pramipexole, ropinirole and pergolide are less efficacious than L-DOPA, particularly in moderate-to-severe PD. However, their side-effect profile is different from that of L-DOPA. It is worth noticing that DA agonists do cause less dyskinesias that L-DOPA but this is of limited value to PD patients with dyskinesias because many of them have moderate-to-severe PD and hence they need the efficacy of L-DOPA

Dyskinesias and other movement disorders from dysfunction of the basal ganglia are of major socio-economic importance. Many attempts have been made to develop agents to prevent and/or treat dyskinesias although such attempts have met with limited success. There is, therefore, a need to provide novel agents to treat dyskinesia.

The 6-hydroxydopamine (6-OHDA) lesion model of parkinsonism in the rat has provided an invaluable tool in the investigation of PD at a preclinical level and for the evaluation of novel therapeutic options (Schwarting and Huston, Prog. Neurobiol. 1996, 50, 275-331). One of the most widely used 6-OHDA paradigms is the evaluation of rotational behavior in rats which bear a discrete degeneration of the dopaminergic nigrostriatal pathway (Ungerstedt and Aburthnott, Brain Res. 1970, 24, 485). In this model, 6-OHDA is unilaterally infused into the nigrostriatal pathway, striatum or medial forebrain bundle (MFB), producing a functional imbalance between the dopaminergic nigrostriatal systems. Administration of drugs directly stimulating dopamine receptors, such as the dopamine metabolic precursor L-DOPA and the dopamine agonist apomorphine produces a rotational behavior directed away from the body side in which 6-OHDA has been infused.

In addition to motor-related deficits, the 6-OHDA model can be used to reproduce other features of PD. The development of both sensitized rotational behavior as well as abnormal involuntary movements (AIMs) has been observed in rats injected with 6-OHDA either in the striatum or in the MFB, and chronically treated with L-DOPA, therefore providing further an animal model for the study of L-DOPA induced dyskinesia (Lundblad, et al. Eur. J Neurosci. 2002, 15,120-132). During chronic treatment in this model, L-DOPA but not bromocriptine induces a gradual development of AIMs. Based on these observations, it has been accepted that rats lesioned with 6-OHDA exhibit motor deficits that share essential functional similarities with Parkinson's dyskinesia and can be used to evaluate the potential of a treatment to provide treatments for dyskinesia.

In an attempt to identify new therapies for treating dyskinesia and other related movement disorders, applicants have surprisingly found that (4aR,10aR)-1-*n*-propyl-1,2,3,4,4a,5,10,10a-octahydro-benzo[g]quinoline-6,7-diol as a potent D1/D2 agonist [herein referred to as Compound **10**]; (6aR,10aR)-7-*n*-propyl-6,6a,7,8,9,10,10a,11-octahydro-1,3-dioxa-7-aza-cyclopenta[a]anthracene [herein referred to as Compound **11**]; and (4aR,10aR)-1-*n*-propyl-2,3,4,4a,5,7,8,9,10,10a-decahydro-1H-benzo[g]quinolin-6-one [herein referred to as Compound **12**] have favorable profiles in rats with unilateral 6-OHDA lesions. They induce less dyskinesias than L-DOPA and apomorphine, and reduce L-DOPA induced dyskinesias more effectively than D2 agonists, as exemplified by pramipexole. Hence, Compounds **10, 11 and 12** have the potential to become the first PD drugs with L-DOPA-like efficacy and a favorable profile not only in terms of both induction of dyskinesia, but also as a medication for the reversal of dyskinesias.

Accordingly, it is expected that above identified compounds can be used to treat dyskinesias and other related movement disorders such as Huntington's chorea. Moreover, the present invention contemplates the use of the corresponding racemic *trans* mixture. The present invention further provides methods of treating Parkinson's disease with a low dyskinesia induction profile comprising administering a therapeutically effective amount of said compound. In one aspect, the treatment of Parkinson's disease is as efficacious as L-DOPA treatment. Further provided are methods of reversing dyskinesias or treating Parkinson's disease comprising administering said compound and pharmaceutical compositions thereof.

WO 2009/026934 discloses compounds 10, 11 and 12 for use in the treatment of neurodegenerative diseases such as PD and Huntington's disease and for use in the treatment of other indications such as dyskinetic disorders, cognitive impairment and restless legs syndrome (RLS).

### SUMMARY OF THE INVENTION

The invention relates to(6aR,10aR)-7-n-propyl-6,6a,7,8,9,10,10a,11-octahydro-1,3-dioxa-7-aza-cyclopenta[a]anthracene or a pharmaceutically acceptable salt thereof, for use in reversing dyskinesias, wherein the dyskinesia is associated with dopamine replacement therapy, wherein the dopamine replacement therapy agent is selected from the group consisting of rotigotine, ropinirole, pramipexole, cabergoline, bromocriptine, lisuride, pergolide, L-DOPA and apomorphine.

In a further embodiment, the invention relates to (6aR,10aR)-7-n-propyl-6,6a,7,8,9,10,10a,11-octahydro-1,3-dioxa-7-aza-cyclopenta[a]anthracene or a pharmaceutically acceptable salt thereof, for use in reversing dyskinesias, wherein the dyskinesia is associated with dopamine replacement therapy, wherein the dopamine replacement therapy agent is selected from the group consisting of rotigotine, ropinirole, pramipexole, cabergoline, bromocriptine, lisuride, pergolide, L-DOPA and apomorphine; wherein the dyskinesia is established as a result of repeated administration of L-DOPA.

### DETAILED DESCRIPTION

The compounds of the present invention contain two chiral centers (denoted with * in the below formula)

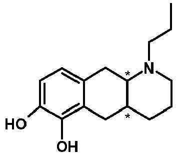

The compounds of the invention can exist in two different diastereomeric forms, the *cis-* and *trans*-isomers, both of which can exist in two enantiomeric forms. The present invention relates only to the *trans* racemate and the (4aR, 10aR)-enantiomer.

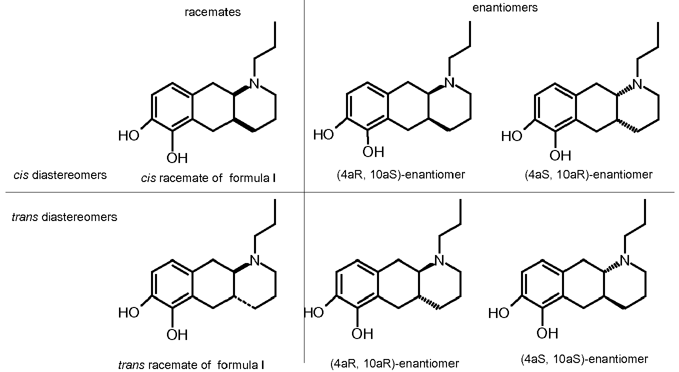

As previously indicated, the present invention is based on the discovery that (4aR,10aR)-1-*n*-propyl-1,2,3,4,4a,5,10,10a-octahydro-benzo[g]quinoline-6,7-diol (herein referred to as "Compound **10**") reversed dyskinesias induced by L-DOPA/benserazide and apomorphine in rats lesioned with 6-OHDA.

Additionally, the compound of the present invention contains two chiral centers (denoted with * in the below formula)

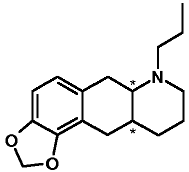

The compound of the invention can exist in two different diastereomeric forms, the *cis-* and *trans*-isomers, both of which can exist in two enantiomeric forms. The present invention relates only to the *trans* racemate and the (6aR, 10aR)-enantiomer.

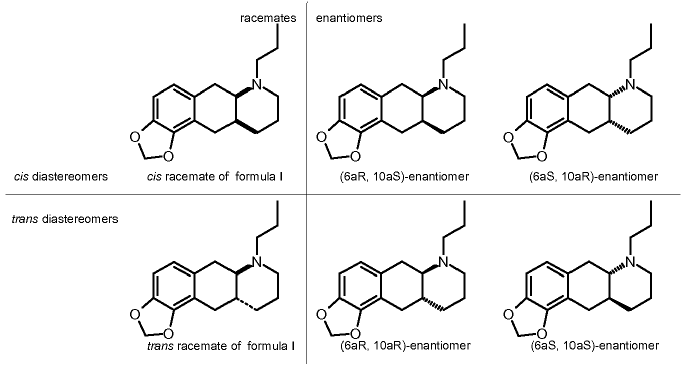

As previously indicated, the present invention is based on the discovery that (6aR,10aR)-7-*n*-propyl-6,6a,7,8,9,10,10a,11-octahydro-1,3-dioxa-7-aza-cyclopenta[a]anthracene (herein referred to as "Compound **11**") reversed dyskinesias induced by L-DOPA/benserazide and apomorphine in rats lesioned with 6-OHDA.

Furthermore, the present invention is based on the discovery that (4aR,10aR)-1-*n-*propyl-2,3,4,4a,5,7,8,9,10,10a-decahydro-1H-benzo[g]quinolin-6-one (herein referred to as Compound **12**) has favorable profiles in rats with unilateral 6-OHDA lesions. It induces less dyskinesias than L-DOPA and apomorphine, and reduces L-DOPA induced dyskinesias more effectively than D2 agonists, as exemplified by pramipexole.

The invention is explained in greater detail below but this description is not intended to be a detailed catalog of all the different ways in which the invention may be implemented, or all the features that may be added to the instant invention.

### Definitions

As used herein, "dyskinesia" refers to a condition characterized by abnormal involuntary movements that are associated with disorders of brain regions known as the basal ganglia. The dyskinesia may be an "L-DOPA-induced dyskinesia" that arises and is a complication of the treatment of Parkinson's disease (the most common basal ganglia disease). Dyskinesia can physically manifest in two forms, chorea and dystonia. Chorea consists of involuntary, continuous, purposeless, abrupt, rapid, brief, unsustained and irregular movements that flow from one part of the body to another. Dystonia refers to sustained muscle contractions that cause twisting and repetitive movements or abnormal postures.

"Treating" or "treatment" refers to inhibiting the disease or disorder, either physically, (e.g., stabilization of a discernible symptom), physiologically, (e.g., stabilization of a physical parameter), or both, and inhibit at least one physical parameter which may not be discernible to the patient. Further, "treating" or "treatment" refers to delaying the onset of the disease or disorder or at least symptoms thereof in a patient which may be exposed to or predisposed to a disease or disorder even though that patient does not yet experience or display symptoms of the disease or disorder.

"Therapeutically effective amount" refers to the amount of a compound that, when administered to a patient for treating a disease or disorder, is sufficient to affect such treatment for the disease or disorder. The "therapeutically effective amount" will vary depending on the compound, the disease or disorder and its severity and the age and weight of the patient to be treated.

As used herein, the phrase "while maintaining a low dyskinesia profile" refers to the dyskinesia profile as seen in patients who have been treated via continuous dopaminergic stimulation. Treatments involving continuous dopaminergic stimulation are described in Stocchi and Olanow, Neurology 2004, 2004, 62, S56-S63; and Hilary, et al., Journal of Neurology 2004,251,11,1370-1374.

As used herein, (4aR,10aR)-1-*n*-propyl-1,2,3,4,4a,5,10,10a-octahydro-benzo[g]quinoline-6,7-diol as a potent D1/D2 agonist is referred to as Compound **10**.

As used herein, (6aR,10aR)-7-*n*-propyl-6,6a,7,8,9,10,10a,11-octahydro-1,3-dioxa-7-aza-cyclopenta[a]anthracene is referred to as Compound **11**.

As used herein, (4aR,10aR)-1-*n*-propyl-2,3,4,4a,5,7,8,9,10,10a-decahydro-1H-benzo[g]quinolin-6-one [herein referred to as Compound **12**.

Compound **10, 11 or 12** may be used to treat dyskinesia as a monotherapy (i.e. use of the compound alone); as an adjunct to compositions to prevent dyskinetic side-effects caused by the composition (e.g. as an adjunct to L-DOPA or apomorphine given to treat parkinsonian patients) or alternatively the compound may be given in combination with other treatments which also reduce dyskinesia (e.g. opioid receptor antagonists, (α2-adrenoreceptor- antagonists, cannabinoid CBI-antagonists, NMDA receptor-antagonists, cholinergic receptor antagonists, histamine H3-receptor agonists, and globus pallidus/subthalamic nucleus lesion/deep brain stimulation).

The present invention is further concerned with the concurrent, separate or sequential use in the treatment of Parkinson's disease while reducing dyskinesia induced by L-DOPA or a dopamine agonist comprising administering a therapeutically effective amount of Compound **11** or a pharmaceutically salt thereof.

In one embodiment, the dyskinesia is associated with a basal ganglia-related movement disorder.

In another embodiment, the dyskinesia is associated with Parkinson's disease.

One embodiment relates to dyskinesia associated with idiopathic Parkinson's disease or post-encephalitic Parkinsonism.

In one embodiment, the dyskinesia is associated with off-dystonia in Parkinson's disease.

In a separate embodiment, the dyskinesia arises as a side-effect of a therapeutic agent to treat Parkinson's disease.

In yet another embodiment, the dyskinesia is associated with dopamine replacement therapy. In one embodiment, dopamine replacement therapy agent is selected from the group consisting of rotigotine, ropinirole, pramipexole, cabergoline, bromocriptine, lisuride, pergolide, L-DOPA and apomorphine.

In one embodiment, the dyskinesia is established as a result of repeated administration of L-DOPA.

As previously indicated, the present invention provides for a pharmaceutical composition comprising Compound **11** or a pharmaceutically acceptable salt thereof, in the preparation of a medicament for treating Parkinson's disease while maintaining a low dyskinesia induction profile, and to a pharmaceutical composition comprising racemic *trans*-1-*n-*propyl-1,2,3,4,4a,5,10,10a-octahydro-benzo[g]quinoline-6,7-diol in the preparation of a medicament for treating Parkinson's disease while maintaining a low dyskinesia induction profile.

In one embodiment, the pharmaceutical composition additionally comprises a MAO-B inhibitor.

In a one embodiment, the MAO-B inhibitor is selegine. In a separate embodiment, the MAO-B inhibitor is rasagiline.

In another embodiment, the invention relates to a pharmaceutical composition comprising a therapeutically effective amount of Compound **11**, or a pharmaceutically acceptable acid addition salt thereof, and one or more pharmaceutically acceptable carriers, diluents and excipients.

In a specific embodiment of the invention, the mammal is a human subject.

The therapeutically effective amount of Compound **11**, calculated as the daily dose of Compound **11** above as the free base, is suitably between 0.01 and 125 mg/day, more suitable between 0.05 and 100 mg/day, e.g. preferably between 0.1 and 50 mg/day.

In a specific embodiment, the daily dose of Compound **11** is between 1.0 and 10 mg/day.

In another embodiment, the daily dose of Compound **11** is less than about 1.0 mg/day.

In a separate embodiment, the daily dose of Compound **11** is about 0.10 mg/day.

In a further embodiment, the invention provides an oral formulation comprising from 0.001 mg to 125 mg of Compound **11**.

In a further embodiment, the invention provides an oral formulation comprising from 0.001 mg to 0.100 mg of Compound **11**.

In a further embodiment, the invention provides an oral formulation comprising from 0.01 mg to 1.0 mg of Compound **11**.

In a further embodiment, the invention provides an oral formulation comprising from 0.10 mg to 10 mg of Compound **11**.

### Pharmaceutically Acceptable Salts

Compound **10, 11 or 12** forms pharmaceutically acceptable acid addition salts with a wide variety of organic and inorganic acids. Such salts of compound 11 are also part of this invention. A pharmaceutically acceptable acid addition salt of Compound **10, 11 or 12** is formed from a pharmaceutically acceptable acid as is well known in the art. Such salts include the pharmaceutically acceptable salts listed in Journal of Pharmaceutical Science, 1977, 66, 2-19 and are known to the skilled person. Typical inorganic acids used to form such salts include hydrochloric, hydrobromic, hydriodic, nitric, sulphuric, phosphoric, hypophosphoric, metaphosphoric, pyrophosphoric, and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include the chloride, bromide, iodide, nitrate, acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, isobutyrate, phenylbutyrate, α-hydroxybutyrate, butyne-1,4-dicarboxylate, hexyne-1,4-dicarboxylate, caprate, caprylate, cinnamate, citrate, formate, fumarate, glycollate, heptanoate, hippurate, lactate, malate, maleate, hydroxymaleate, malonate, mandelate, mesylate, nicotinate, isonicotinate, oxalate, phthalate, teraphthalate, propiolate, propionate, phenylpropionate, salicylate, sebacate, succinate, suberate, benzenesulfonate, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, p-toluenesulfonate, xylenesulfonate, tartrate, and the like.

### Pharmaceutical Compositions

Methods of the preparation of solid pharmaceutical compositions are also well known in the art. Tablets may thus be prepared by mixing the active ingredient with ordinary adjuvants, fillers and diluents and subsequently compressing the mixture in a convenient tabletting machine. Examples of adjuvants, fillers and diluents comprise microcrystalline cellulose, corn starch, potato starch, lactose, mannitol, sorbitol talcum, magnesium stearate, gelatine, lactose, gums, and the like. Any other adjuvant or additive such as colorings, aroma, preservatives, etc. may also be used provided that they are compatible with the active ingredients.

In particular, the tablet formulations according to the invention may be prepared by direct compression of Compound **11** in admixture with conventional adjuvants or diluents. Alternatively, a wet granulate or a melt granulate of Compound **11**, optionally in admixture with conventional adjuvants or diluents may be used for compression of tablets.

Solutions of Compound **11** for injections may be prepared by dissolving the active ingredient and possible additives in a part of the solvent for injection, preferably sterile water, adjusting the solution to the desired volume, sterilization of the solution and filling in suitable ampoules or vials. Any suitable additive conventionally used in the art may be added, such as tonicity agents, preservatives, antioxidants, solubilizing agents, etc.

### BRIEF DESCRIPTION OF THE FIGURES

FIGURE 1: Crystal structure of compound *ent*-**10**. The absolute configuration was determined by the anomalous scattering of the 'heavy' bromine atom.
FIGURE 2: Dose-response curve for the concentration-dependent stimulation of intracellular Ca²⁺ release by dopamine in hD₅-transfected CHO-Ga16 cells.

### EXPERIMENTAL SECTION

Analytical LC/MS data were obtained on a PE Sciex API 150EX instrument equipped with atmospheric pressure photo ionization and a Shimadzu LC-8A/SLC-10A LC system. Purity was determined by integration of the UV (254 nm) and ELSD traces. MS instruments are from Peskier (API), equipped with APPI-source and operated in positive ion mode. The retention times in the UV-trace (RT) are expressed in min. Solvents A was made of 0.05% TFA in water, while solvent B was made of 0.035% TFA and 5% water in acetonitrile. Several different methods have been used:
Method 25: API 150EX and Shimadzu LC10AD/SLC-10A LC system. Column: dC-18 4.6x30mm, 3µm (Atlantis, Waters). Column temperature: 40 °C. Gradient: reverse phase with ion pairing. Flow: 3.3 mL/min. Injection volume: 15 µL. Gradient: 2% B in A to 100% B over 2.4 min then 2% B in A for 0.4 min. Total run time: 2.8 min.
Method 14: API 150EX and Shimadzu LC8/SLC-10A LC system. Column: C-18 4.6x30mm, 3.5µm (Symmetry, Waters). Column temperature: rt. Gradient: reverse phase with ion pairing. Flow: 2mL/min. Injection volume: 10 µL. Gradient: 10% B in A to 100% B over 4 min then 10% B in A for 1 min. Total run time: 5 min.

X-ray crystal structure determination was performed as follows. The crystal of the compound was cooled to 120 K using a Cryostream nitrogen gas cooler system. The data were collected on a Siemens SMART Platform diffractometer with a CCD area sensitive detector. The structures were solved by direct methods and refined by full-matrix least-squares against F² of all data. The hydrogen atoms in the structures could be found in the electron density difference maps. The non-hydrogen atoms were refined anisotropically. All the hydrogen atoms were at calculated positions using a riding model with O-H=0.84, C-H = 0.99-1.00, N-H = 0.92-0.93 Å. For all hydrogen atoms the thermal parameters were fixed [U(H) = 1.2 U for attached atom]. The Flack x-parameters are in the range 0.0(1)-0.05(1), indicating that the absolute structures are correct. Programs used for data collection, data reduction and absorption were SMART, SAINT and SADABS [cf. "SMART and SAINT, Area Detector Control and Integration Software", Version 5.054,Bruker Analytical X-Ray Instruments Inc., Madison, USA (1998), Sheldrick "SADABS, Program for Empirical Correction of Area Detector Data" Version 2.03, University of Göttingen, Germany (2001)]. The program SHELXTL [cf. Sheldrick "SHELXTL, Structure Determination Programs", Version 6.12, Bruker Analytical X-Ray Instruments Inc., Madison, USA (2001)] was used to solve the structures and for molecular graphics.

### Synthesis of the compounds 10 and 11

Starting from compound **1** whose synthesis is described in the literature prepared as described in Taber et al., J. Am. Chem. Soc., 124(42), 12416 (2002), compound **8** can be prepared as described herein in eight steps. This material can be resolved by chiral SFC as described herein to give compounds **9** and *ent*-**9**. After cleavage of the Boc-protective group, reductive amination can be used to introduce the n-propyl group on the nitrogen atom. The resulting masked catechol amines can be deprotected under standard conditions by treatment with 48% HBr or by reaction with BBr₃ to give compounds **10** and *ent*-**10**. Further reaction of **10** with CH₂ClBr or a related reagent in the presence of base can be applied to give compound **11**.

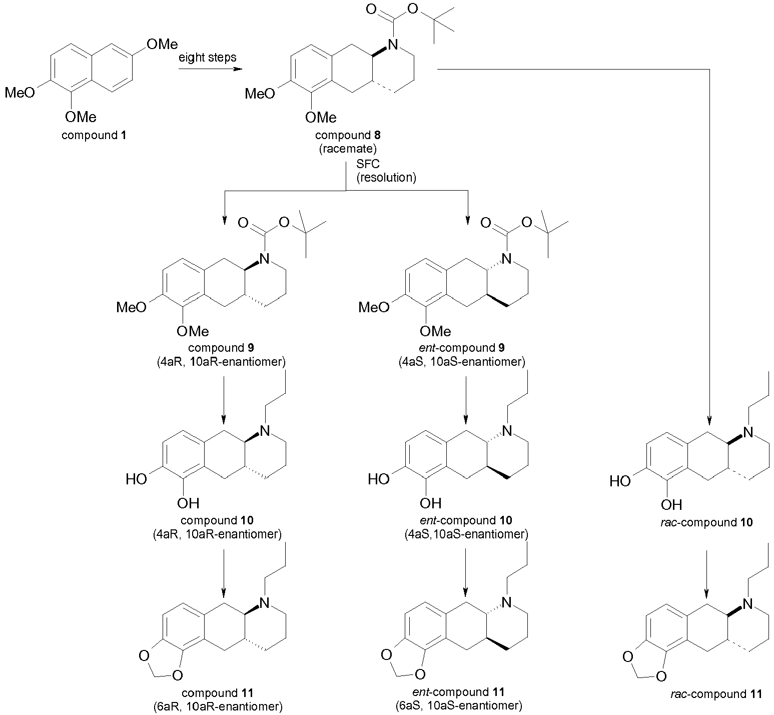

### Synthesis of compounds 10 and ent-10.

### 7-Iodo-1,2,6-trimethoxy-naphthalene (compound 2).

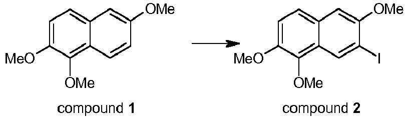

To a stirred solution of compound **1** (26.2 g; prepared as described in Taber et al., J. Am. Chem. Soc., 124(42), 12416 (2002) in dry THF (200 mL) under argon and at -78 °C was slowly added *s*-butyl lithium (1.2 M in cyclohexane, 110 mL). The solution was stirred at - 78°C for 3h. A solution of iodine (30.5 g) in dry THF (50 mL) was added over a period of 10 min. The resulting mixture was then stirred for another 10 min at -78 °C. The reaction mixture was quenched by the addition of sat. NH₄Cl (100 mL), water (240 mL), and Et₂O (240 mL). The organic layer was washed with 10% aqueous sodium sulfite solution (100 mL), dried (Na₂SO₄) and concentrated *in vacuo.* The crude material was purified by distilling off unreacted starting material. The residue was further purified by silica gel chromatography (EtOAc/heptane) to produce an impure solid material, which was purified by precipitation from EtOAc/heptane affording 11.46 g of compound **2**.

### (E/Z)-3-(3,7,8-Trimethoxy-naphthalen-2-yl)-acrylonitrile (compound 3).

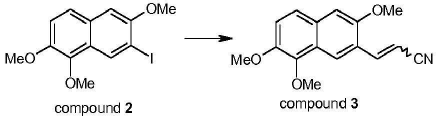

To a suspension of compound **2** (3.41 g) in dry acetonitrile (10.7 mL) in a microwave reactor vial was added acrylonitrile (1.19 mL) Pd(OAc)₂ (73 mg), and triethylamine (1.48 mL). The vial was sealed, and the mixture was heated for 40 min at 145 °C under microwave irradiation. This procedure was carried out two more times (using a total of 10.23g of compound **5**). The crude reaction mixtures were combined and the catalyst was filtered off, and the filtrate was concentrated *in vacuo.* The residue was partitioned between Et₂O (300 mL) and 2M HCl (150 mL). The organic layer was washed with brine (100 mL), dried (Na₂SO₄) and concentrated *in vacuo.* The crude material (7.34 g) was purified by silica gel chromatography (EtOAc/heptane) to produce 5.23 g of compound **3** as a mixture of olefin isomers.

### 3-(3,7,8-Trimethoxy-naphthalen-2-yl)-propionitrile (compound 4).

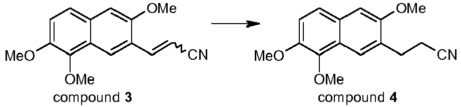

Compound **3** (5.23 g) was dissolved in CHCl₃ (15 mL) and 99% EtOH (100 mL). 10% Pd/C (0.8 g) was added and the solution was hydrogenated for 45 min under a hydrogen pressure of 3 bar using a Parr shaker. The catalyst was filtered off, and the filtrate was passed through a small plough of silica gel (eluent: 99% EtOH). Yield: 4.91 g compound **4** as a white solid.

### [3-(3,7,8-Trimethoxy-1,4-dihydro-naphthalen-2-yl)-propyl]-carbamic acid t-butyl ester (compound 5).

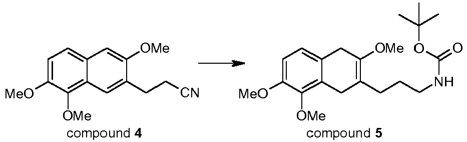

Compound **4** (5.0g) was dissolved in 99% EtOH (150 mL) and the mixture was heated to reflux under nitrogen atmosphere. Sodium metal (5g) was added in small lumps over 3h. The mixture was refluxed for an addition 2h, before it was stirred at rt for 2 days. Then it was heated to reflux again, and more sodium metal (3.68 g) was added and the mixture was refluxed overnight. After cooling on an ice/water bath, the reaction was quenched by the addition of solid ammonium chloride (20 g) and water (25 mL). The resulting mixture was filtered, and the filtrate was concentrated *in vacuo.* The residue was partitioned between diethyl ether (50 mL) and water (50 mL). The aqueous layer was neutralized with 37% HCl and extracted with diethyl ether (2x50 mL). The combined organic extracts were washed with brine (50 mL), dried (MgSO₄) and concentrated *in vacuo* to afford an oil. This material was dissolved in THF (50 mL) and treated with Boc₂O (2.34 g) and Et₃N (1.78 mL) at rt. After six days the volatiles were removed *in vacuo* and the residue was purified by silica gel chromatography (EtOAc/heptane). This provided impure compound **5** (1.52 g).

### Racemic 6,7-dimethoxy-2,3,4,4a,5,10-hexahydro-benzo[g]quinoline hydrochloride (compound 6).

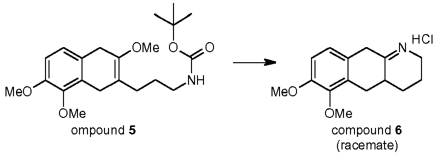

Compound **5** (1.52 g from the previous step) was dissolved in MeOH (20 mL). 37% HCl (3.5 mL) was added, and the mixture was refluxed for 4h. The volatiles were removed *in vacuo*, using toluene to azeotropically remove the water. This provided impure compound **6** (0.89 g) as an yellow oil.

### Racemic trans-6,7-dimethoxy-3,4,4a,5,10,10a-hexahydro-2H-benzo[g]quinoline-1-carboxylic acid t-butyl ester (compound 8).

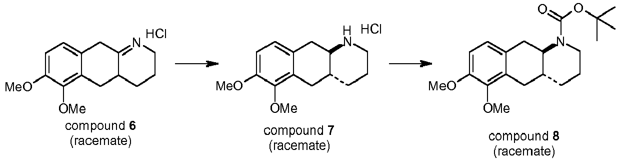

Compound **6** (0.89 g) was dissolved in MeOH (10 mL) and NaCNBH₃ (0.19 g) was added. The reaction was stirred overnight at rt. The crude mixture was cooled on an ice/water bath, before it was quenched with 2 M HCl in Et₂O (1 mL). The mixture was partitioned between Et₂O (50 mL), water (50 mL), and 2 M NaOH (10 mL). The aqueous layer was extracted with diethyl ether (3x50 mL). The combined organic layers were dried (MgSO₄) and concentrated *in vacuo* to afford the impure free amine (compound **7**). This material was dissolved in THF (25 mL) and treated with Boc₂O (0.68 g) and Et₃N (0.86 mL) at rt for 1h. The crude mixture was concentrated *in vacuo,* and the residue was purified by silica gel chromatography (EtOAc/heptane) to provide 1.18g of slightly impure racemic compound **8.**

### SFC-separation of the enantiomers of racemic trans-6,7-dimethoxy-3,4,4a,5,10,10a-hexahydro-2H-benzo[g]quinoline-1-carboxylic acid t-butyl ester (compounds 9 and ent-9).

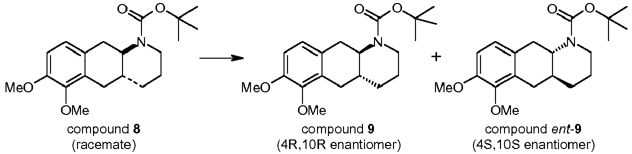

Compound **8** (19.7 g) was resolved into its enantiomers using chiral SFC on a Berger SFC multigram II instrument equipped with a Chiralcel OD 21.2 x 250 mm column. Solvent system: CO₂/EtOH (85:15), Method: constant gradient with a flow rate of 50 mL/min. Fraction collection was performed by UV 230 nm detection. Fast eluting enantiomer (4aR, 10aR enantiomer; compound **9**): 9.0 g of a white solid. Slow eluting enantiomer (4aS, 10aS enantiomer; compound *ent*-**9**): 8.1 g of a white solid.

### (4aS,10aS)-6,7-Dimethoxy-1,2,3,4,4a,5,10,10a-octahydro-benzo[g]quinoline hydrochloride (compound ent-9').

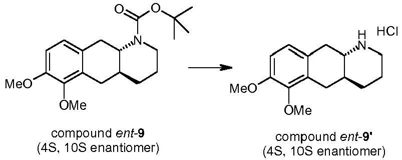

Compound *ent*-**9** (0.52g) was dissolved in MeOH (15 mL) and treated with 5 M HCl in Et₂O (7.5 mL) at rt for 2h.. The mixture was concentrated *in vacuo* and the solid was dried *in vacuo* to give compound *ent*-**9**' as a white solid. LC/MS (method 14): RT 1.31 min.

### (4aR,10aR)-1-Propyl-1,2,3,4,4a,5,10,10a-octahydro-benzo[g]quinoline-6,7-diol hydrobromide (compound 10).

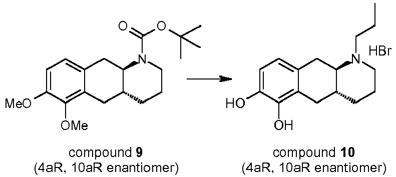

Compound 9 (0.5 g) was dissolved in 99% EtOH (5 mL) and treated with 2M HCl in Et₂O (4 mL) overnight at rt. The crude mixture was concentrated *in vacuo,* and the residue was partitioned between EtOAc and 10% aqueous NaOH (5 mL). The aqueous layer was extracted with EtOAc, and the combined organic layers were washed with brine, dried (MgSO₄), concentrated *in vacuo.* The residue was dissolved in 99% EtOH (5 mL) and treated with propionic aldehyde (0.52 mL), NaCNBH₃ (0.45 g), and AcOH (3 drops) overnight at rt. The crude mixture was portioned between sat. aqueous NaHCO₃ (12.5 mL), water (12.5 mL), and EtOAc (2x25 mL). The combined organic layers were washed with brine, dried (MgSO₄), and concentrated *in vacuo.* The residue was purified by silica gel chromatography (MeOH/EtOAc). The obtained intermediate was treated with 48% HBr (3 mL) at 150 °C for 1h under microwave conditions, before the crude mixture was stored at 4 °C overnight. The precipitated material was isolated by filtration and dried *in vacuo.* Yield of compound **10:** 103 mg as a solid. LC/MS (method 25): RT 0.77 min.

### (4aS,10aS)-1-Propyl-1,2,3,4,4a,5,10,10a-octahydro-benzo[g]quinoline-6,7-diol hydrobromide (compound ent-10).

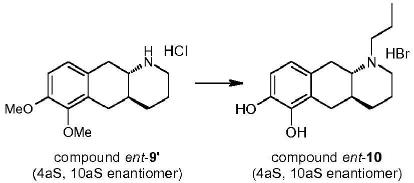

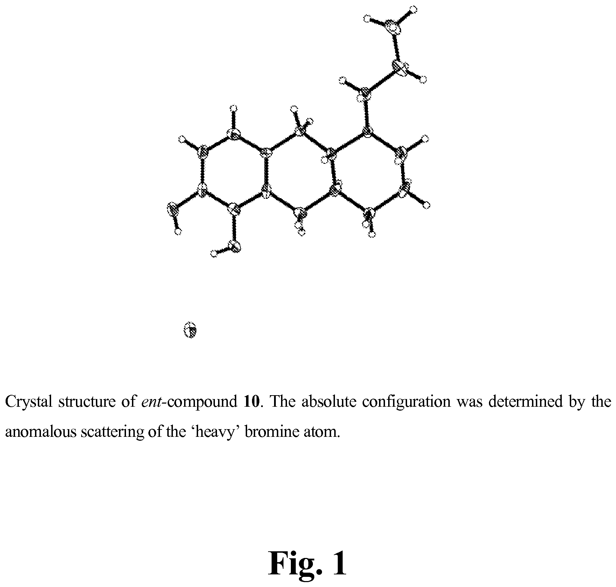

The procedure described for compound **10** was followed starting from compound *ent*-**9**' (0.5 g; the HCl salt was liberated by partitioning between EtOAc and 10% aqueous NaOH before the reductive amination step). Yield of compound *ent*-**10**: 70 mg as a solid. LC/MS (method 25): RT 0.70 min. A small sample of compound *ent*-**10** was dissolved in MeOH and allowed to crystallize slowly at rt over 2 months. The formed white crystals were collected and subjected to X-ray analysis (cf. Figure 1). The absolute configuration of compound *ent*-**10** was determined by X-ray crystallography and allowed for unambiguous determination of the stereochemistry of compounds **9** and **10** and hence their derivatives.

### (6aR,10aR)-7-n-Propyl-6,6a,7,8,9,10,10a,11-octahydro-1,3-dioxa-7-aza-cyclopenta[a]anthracene hydrochloride (compound 11).

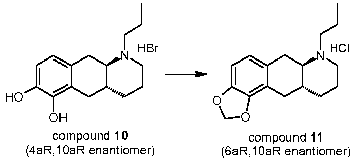

Compound **10** (7.80 g), Cs₂CO₃ (18.6 g), CH₂BrCl (2.2 mL), and DMF (180 mL) were heated to 100 °C for 1h under an argon atmosphere. The crude reaction mixture was added to separatory funnel and diluted with ice/water (300 mL). The resulting mixture was extracted with Et₂O (3x300 mL). The combined organic layers were washed with brine (200 mL), dried (MgSO₄) and concentrated *in vacuo.* The residue was purified by silica gel chromatography (EtOAc/MeOH) to afford a pale red solid, which was dissolved in MeOH (25 mL) and precipitated as the hydrochloride salt by addition of 2 M HCl in Et₂O (20 mL) and Et₂O (100 mL). The precipitated product was isolated by filtration and dried *in vacuo.* Yield of compound **11:** 5.1 g. LC/MS (method 111): RT 0.70 min. ELSD 100%. UV 97.0%. MH⁺: 274.0.

### (4aR,10aR)-n-1-propyl-2,3,4,4a,5,7,8,9,10,10a-decahydro-1H-benzo[g]quinolin-6-one (Compound 12)

The synthesis of Compound **12** can be prepared as described in EP Patent No. 1274411. Compound **12** is referred to as (-)-GMC6650 in the above-identified patent. Compound 12 is included as a comparative example and does not form part of the invention.

### EXPERIMENTAL SECTION

### Example 1: Compounds 11 and 12 convert into the catechol-containing active metabolite of compound 10 upon in-vivo administration.

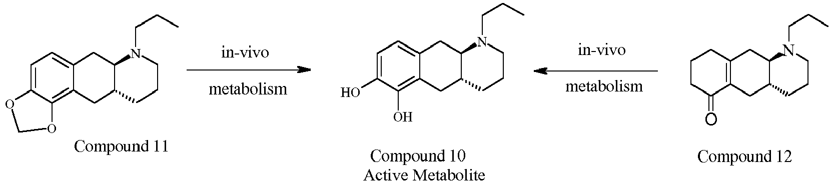

The active metabolite (i.e. Compound **10**) was found to function as a potent agonist at both the D1 and D2 receptors *in-vitro.* As discussed in greater detail below, the data generated from *in-vivo* experiments indicate that this active metabolite possesses a superior profile against other dopamine agonists and is on par with the efficacy seen with L-DOPA/apomorphine treatment.

### Example 2: Pharmacological Testing of Compound 10

### D₁ cAMP assay

The ability of the compounds to either stimulate or inhibit the D₁ receptor mediated cAMP formation in CHO cells stably expressing the human recombinant D₁ receptor was measured as follows. Cells were seeded in 96-well plates at a concentration of 11000 cells/well 3 days prior to the experiment. On the day of the experiment the cells were washed once in preheated G buffer (1 mM MgCl₂, 0.9 mM CaCl₂, 1 mM IBMX (3-*i*-butyl-1-methylxanthine) in PBS (phosphate buffered saline)) and the assay was initiated by addition of 100 micro-L of a mixture of 30 nM A68930 and test compound diluted in G buffer (antagonism) or test compound diluted in G buffer (agonism).

The cells were incubated for 20 minutes at 37 °C and the reaction was stopped by the addition of 100 micro-L S buffer (0.1 M HCl and 0.1 mM CaCl₂) and the plates were placed at 4 °C for 1h. 68 micro-L N buffer (0.15 M NaOH and 60 mM NaOAc) was added and the plates were shaken for 10 minutes. 60 micro-1 of the reaction were transferred to cAMP FlashPlates (DuPont NEN) containing 40 micro-L 60 mM Sodium acetate pH 6.2 and 100 micro-L IC mix (50 mM Sodium acetate pH 6.2, 0.1 % sodium azide, 12 mM CaCl₂, 1% BSA (bovine serum albumin) and 0.15 micro-Ci/mL ^{12S}I-cAMP) were added. Following an 18h incubation at 4 °C the plates were washed once and counted in a Wallac TriLux counter. Compound **10** was demonstrated to act as a D₁ agonist in this assay.

### D₂ cAMP assay

The ability of the compounds to either stimulate or inhibit the D₂ receptor mediated inhibition of cAMP formation in CHO cells transfected with the human D₂ receptor was measure as follows. Cells were seeded in 96 well plates at a concentration of 8000 cells/well 3 days prior to the experiment. On the day of the experiment the cells were washed once in preheated G buffer (1 mM MgCl₂, 0.9 mM CaCl₂, 1 mM IBMX in PBS) and the assay was initiated by addition of 100 micro-1 of a mixture of 1 micro-M quinpirole, 10 microM forskolin and test compound in G buffer (antagonism) or 10 micro-M forskolin and test compound in G buffer (agonism).

The cells were incubated 20 minutes at 37 °C and the reaction was stopped by the addition of 100 micro-1 S buffer (0.1 M HCl and 0.1 mM CaCl₂) and the plates were placed at 4 °C for 1h. 68 micro-L N buffer (0.15 M NaOH and 60 mM Sodium acetate) were added and the plates were shaken for 10 minutes. 60 micro-L of the reaction were transferred to cAMP FlashPlates (DuPont NEN) containing 40 micro-L 60 mM NaOAc pH 6.2 and 100 micro-L IC mix (50 mM NaOAc pH 6.2, 0.1 % Sodium azide, 12 mM CaCl₂, 1% BSA and 0.15 micro-Ci/ml ¹²⁵I-cAMP) were added. Following an 18h incubation at 4 °C the plates were washed once and counted in a Wallac TriLux counter. Compound **10** was demonstrated to act as a D₂ agonist in this assay.

### D₅ assay

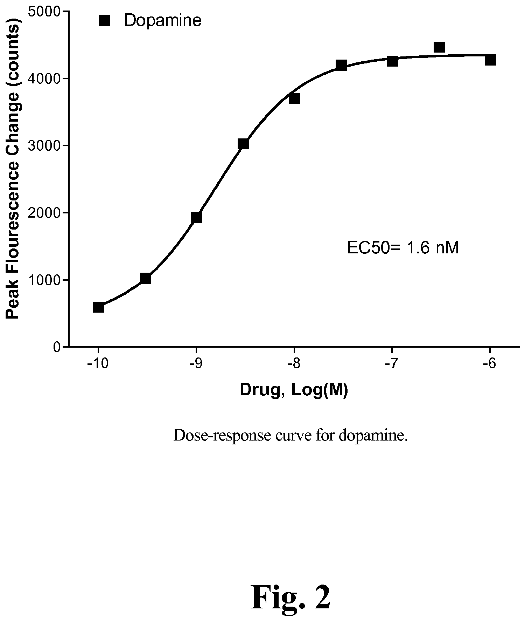

Concentration-dependent stimulation of intracellular Ca²⁺ release by dopamine in hD₅-transfected CHO-Ga16 cells. The cells were loaded with fluoro-4, a calcium indicator dye, for 1h. Calcium response (fluorescence change) was monitored by FLIPR (fluorometric imaging plate reader) for 2.5 min. Peak responses (EC₅₀) were averaged from duplicate wells for each data point and plotted with drug concentrations (cf. Figure 2 for dopamine). Compound **10** was demonstrated to act as a D₅ agonist in this assay.

### 6-OHDA Rat Model

Dopamine agonists can have activity at either the D1 receptors, the D2 receptors, or both. The rotation response in rats with unilateral 6-OHDA lesions can be used to assess compounds for their ability to stimulate both receptor types and induce rotation (Ungerstedt and Arbuthnott, Brain Res., 1970, 24, 485; Setler, et al. Eur. J. Pharmacol., 1978, 50(4), 419; and Ungerstedt, et al. "Advances in Dopamine Research" (Kohsaka, Ed.), Pergamon Press, 1982, Oxford, p. 219). 6-OHDA (6-hydroxydopamine) is a neurotoxin used by neurobiologists to selectively kill dopaminergic neurons at the site of injection in the brain in experimental animals. In the 6-OHDA model, the nigrostraital dopamine cells are destroyed on one side of the brain (unilateral) by injecting 6-OHDA into the median forebrain bundle, located in front of the substantia nigra. This unilateral injection combined with stimulation by dopamine agonists such as apomorphine will induce rotation behaviour as only one side of the brain is stimulated. Experiments consist of determining a minimum effective dose (MED) to induce rotation for the compound in question. Once a MED has been determined, a second experiment is performed to determine the MED of the compound to overcome Nemonapride block (MED_{Nemonapride}). Nemonapride is a D2 antagonist that blocks the D2 receptor, therefore any observed rotations would be dependent upon activity at the D1 receptor. Finally, once the MED_{Nemonapride} is known a third experiment is run using the MED_{Nemonapride} dose and observing the effect of the D1 antagonist, SCH 23390 alone, the D2 antagonist, Nemonapride alone and finally, the effect of combined treatment with SCH 23390 and Nemonapride. This third experiment confirms the activity of the compound at both receptors as either antagonist alone can only partially inhibit the rotation response induced by the test compound while the combination treatment completely blocks all rotations in the rats [Arnt and Hyttel, Psychopharmacology, 1985, 85(3), 346; and Sonsalla et al., J. Pharmacol Exp. Ther., 1988, 247(1), 180]. This model was validated using apomorphine as the proof-of-principle compound for mixed D1/D2 agonists.

In this model, Compound **10** possess 'apomorphine'-like profiles with a D1/D2 ratio of about 2-4 as compared to a ratio of about 3 for apomorphine. Moreover, the duration of action observed was ca. 18h for the compound which is significantly higher than that seen with L-DOPA / apomorphine. A D1 component could not be observed for D2-agonists as exemplified by pramipexole and rotigotine.

### Superiority model

Apomorphine and L-DOPA are able to reverse motility deficits in a mouse model of severe dopamine depletion. Both Apomorphine and L-DOPA stimulate D1 and D2 dopamine receptors. Pramipexole, an agonist at D2 receptors is ineffective in this model.

The experiments were performed as follows: Mice previously treated with MPTP (2x15mg/kg subcutaneously) and that had stable lesions are used and vehicle treated mice served as normal controls. MPTP (1-methyl-4-phenyl-1,2,3,6-tetrahydropyridine) is a neurotoxin that causes permanent symptoms of Parkinson's disease by killing certain neurons in the substantia nigra of the brain. It is used to study the disease in monkeys and mice. On the day of the experiment, mice were treated with AMPT (250mg/kg subcutaneously) and then returned to their home cages for 1.5 hours after which they were placed in individual cages in the motility unit. AMPT (alpha-methyl-p-tyrosine) is a drug that temporarily reduces brain catecholamine activity (in this case especially dopamine levels). Three hours after the AMPT injection, rescue of locomotive deficits is attempted with Compound **10** and activity was recorded for an additional 1.5 hours. The first 30 min of data collected after the rescue treatment was 'contaminated' due to stressing the animals with handling and injection as evidenced by increased levels in the vehicle controls therefore the data were analyzed using the last 1 hour of recorded data. Various dopaminergic compounds were tested for their ability to reverse the motility deficits produced in this model. Both L-DOPA/Benserazide, and apomorphine restored locomotion in the mice in a dose-dependent manner. Benserazide is a DOPA decarboxylase inhibitor which is unable to cross the blood-brain barrier; it is used to prevent metabolism of L-DOPA to dopamine outside the brain. In contrast, the D2 agonists, pramipexole and bromocriptine did not restore the locomotion in the mice.

This model was used to evaluate whether or not Compound 10 exhibits the same superiority as L-DOPA and apomorphine over D2 agonists. A dose response experiment for Compound **10** was performed and there was a dose-dependent trend for reversing the hypomotility deficits induced by severe depletion of endogenous dopamine. A final experiment directly comparing the effects of apomorphine, pramipexole and Compound **10** in this model was performed and confirmed that Compound **10** was able to restore locomotion in MPTP mice treated and was superior to pramipexole.

### Induction of dyskinesia model with naive 6-OHDA rats

Twenty male Sprague Dawley rats with unilateral 6-OHDA lesions were used to test induction of dyskinesia by compound **10** (administered subcutaneously; n=7; group 1) compared to L-DOPA/benserazide (6mg/kg / 15mg/kg subcutaneously; n=7; group 2) and apomorphine (1mg/kg subcutaneously; n=6; group 3). Benserazide is a DOPA decarboxylase inhibitor which is unable to cross the blood-brain barrier; it is used to prevent metabolism of L-DOPA to dopamine outside the brain. Three weeks after 6-OHDA surgery, the animals were tested for their rotation response induced by 2.5mg/kg amphetamine, which induces ipsilateral circling (amphetamine increases the level dopamine in the brain via the intact neurons on the unlesioned side causing the animals to rotate in the opposite direction as compared to their response to direct agonists such as L-DOPA and apomorphine that act predominantly on the lesioned side of the brain). All animals included in this study met the criteria of greater than 350 rotations in 60 min. Rats where then randomly allocated to the three treatment groups balancing the groups for the animals' rotation response on amphetamine.

During the actual dyskinesia experiments, rats received once daily injections of the test compounds subcutaneously and were observed for 3h following injection. Each animal was observed for 1 minute every 20 min throughout the 3h period for the presence of dyskinesias using the Abnormal Involuntary Movement Scale (AIMS) as described previously (Lundblad, et al., Eur. J Neurosci., 15, 120, (2002)). Rats received drug for 14 consecutive days and were scored on days 1, 2, 3, 4, 5, 8, 10 and 12. Two-way repeated measures ANOVA revealed that there was a significant treatment effect, time effect and treatment by time interaction (p<0.001, in all cases). Post hoc comparisons using Holm-Sidak method indicates that animals treated with compound **10** had significantly less dykinesia (scores of about 30) compared to animals treated with either L-DOPA or apomorphine (scores of about 70). There were no differences between L-DOPA and apomorphine treated groups. Following this experiment all rats were given subcutaneous injections of compound **10** from day 15-19 in order to determine how Example I influenced the severity of dyskinesia seen in the apomorphine and L-DOPA groups. Dykinesia scoring was performed on day 19 of the experiment (corresponding to 5 days on compound **10**). The data showed a partial reversal of the dyskinesias induced by L-DOPA and apomorphine to about the level of dyskinesias induced by compound **10** (which did not cause an increase in dyskinesia in group 1 as compared to the score of about 30 observed after 12 days of treatment).

### Dyskinesia Rat Model

A separate dyskinesia study addressed the reversal of L-DOPA induced dyskinesias with either pramipexole or compound **10.** Briefly, 18 animals were treated with L-DOPA/Benserazide (6/15mg/kg subcutaneously) for 7 days. Animals were observed on Days 1, 3 and 5 and AIMS were scored. The day 5 scores were then used to separate the animals into three groups of 6 animals each. Group 1 continued with daily L-DOPA treatment. Group 2 was treated with compound **10** (administered subcutaneously). Group 3 was treated with pramipexole (0.16mg/kg subcutaneously). Treatment continued daily for 10 days and the amount of dyskinesia was scored on days 1, 5, 9 and 10. Two-way repeated measures analysis of variance indicates that animals treated with compound **10** had significantly fewer dyskinesias than both the pramipexole group and the L-DOPA/Benserazide group. The pramipexole group had significantly less dyskinesias than the L-DOPA/Benserazide group. Hence, compound **10** had a superior profile over pramipexole in terms of reversing dyskinesias induced by L-DOPA.

### Anti-Parkinsonian effects in MPTP-treated common marmosets

The experiments were conducted using 6 MPTP treated marmosets (2.0mg/kg daily for up to 5 consecutive days dissolved in sterile 0.9% saline solution). All the animals had previously been treated with L-DOPA (12.5mg/kg p.o., plus carbidopa 12.5mg/kg p.o.) administered daily for up to 30 days in order to induce dyskinesia. Prior to the study all subjects exhibited stable motor deficits including a marked reduction of basal locomotor activity, poor coordination of movement, abnormal and/or rigid posture, reduced alertness and head checking movements. Domperidone was administered 60 min before any of the test compounds. Domperidone is an anti-dopaminergic drug that suppresses nausea and vomiting. Locomotor Activity was assessed using test cages that are comprised of 8 photo-electric switches comprised of 8 infra-red beams which are strategically placed in the cage and interruption of a beam is recorded as one count. The total number of beam counts per time segment is then plotted as time course or displayed as area under the curve (AUC) for total activity. The assessment of motor disability was performed by a trained observer blinded to the treatment.

L-DOPA (12.5mg/kg, p.o.) increased locomotor activity and reversed motor disability as previously described (Smith, et al. Mov. Disord. 2002, 17(5), 887). The dose chosen for this challenge is at the top of the dose response curve for this drug. Compound **10** (dosed subcutaneously) produced dose-related increases in locomotor activity and reversal of motor disability tending to produce in a response greater than for L-DOPA (12.5mg/kg, p.o.). Both test compounds produced a prolonged reversal of motor disability compared to L-DOPA and were as efficacious as L-DOPA. Compound **10** produced a prolonged reversal of motor disability compared to L-DOPA and was as efficacious as L-DOPA.

### Example 3: Pharmacological Testing of Compound 11

### D₁ cAMP assay

The ability of the compounds to either stimulate or inhibit the D₁ receptor mediated cAMP formation in CHO cells stably expressing the human recombinant D₁ receptor was measured as follows. Cells were seeded in 96-well plates at a concentration of 11000 cells/well 3 days prior to the experiment. On the day of the experiment the cells were washed once in preheated G buffer (1 mM MgCl₂, 0.9 mM CaCl₂, 1 mM IBMX (3-*i*-butyl-1-methylxanthine) in PBS (phosphate buffered saline)) and the assay was initiated by addition of 100 micro-L of a mixture of 30 nM A68930 and test compound diluted in G buffer (antagonism) or test compound diluted in G buffer (agonism).

The cells were incubated for 20 minutes at 37 °C and the reaction was stopped by the addition of 100 micro-L S buffer (0.1 M HCl and 0.1 mM CaCl₂) and the plates were placed at 4 °C for 1h. 68 micro-L N buffer (0.15 M NaOH and 60 mM NaOAc) was added and the plates were shaken for 10 minutes. 60 micro-1 of the reaction were transferred to cAMP FlashPlates (DuPont NEN) containing 40 micro-L 60 mM Sodium acetate pH 6.2 and 100 micro-L IC mix (50 mM Sodium acetate pH 6.2, 0.1 % sodium azide, 12 mM CaCl₂, 1% BSA (bovine serum albumin) and 0.15 micro-Ci/mL ^{12S}I-cAMP) were added. Following an 18h incubation at 4 °C the plates were washed once and counted in a Wallac TriLux counter. The active metabolite or Compound **10** was found to be a D₁ agonist in this assay.

### D₂ cAMP assay

The ability of the compounds to either stimulate or inhibit the D₂ receptor mediated inhibition of cAMP formation in CHO cells transfected with the human D₂ receptor was measure as follows. Cells were seeded in 96 well plates at a concentration of 8000 cells/well 3 days prior to the experiment. On the day of the experiment the cells were washed once in preheated G buffer (1 mM MgCl₂, 0.9 mM CaCl₂, 1 mM IBMX in PBS) and the assay was initiated by addition of 100 micro-1 of a mixture of 1 micro-M quinpirole, 10 microM forskolin and test compound in G buffer (antagonism) or 10 micro-M forskolin and test compound in G buffer (agonism).

The cells were incubated 20 minutes at 37 °C and the reaction was stopped by the addition of 100 micro-1S buffer (0.1 M HCl and 0.1 mM CaCl₂) and the plates were placed at 4 °C for 1h. 68 micro-L N buffer (0.15 M NaOH and 60 mM Sodium acetate) were added and the plates were shaken for 10 minutes. 60 micro-L of the reaction were transferred to cAMP FlashPlates (DuPont NEN) containing 40 micro-L 60 mM NaOAc pH 6.2 and 100 micro-L IC mix (50 mM NaOAc pH 6.2, 0.1 % Sodium azide, 12 mM CaCl₂, 1% BSA and 0.15 micro-Ci/ml ¹²⁵I-cAMP) were added. Following an 18h incubation at 4 °C the plates were washed once and counted in a Wallac TriLux counter. The active metabolite or Compound **10** was found to be a D₂ agonist in this assay.

### D5 assay

Concentration-dependent stimulation of intracellular Ca²⁺ release by dopamine in hD₅-transfected CHO-Ga16 cells. The cells were loaded with fluoro-4, a calcium indicator dye, for 1h. Calcium response (fluorescence change) was monitored by FLIPR (fluorometric imaging plate reader) for 2.5 min. Peak responses (EC₅₀) were averaged from duplicate wells for each data point and plotted with drug concentrations. The active metabolite or Compound **10** was found to be a D₅ agonist in this assay.

### 6-OHDA Rat Model

Dopamine agonists can have activity at either the D1 receptors, the D2 receptors, or both. The rotation response in rats with unilateral 6-OHDA lesions can be used to assess compounds for their ability to stimulate both receptor types and induce rotation (Ungerstedt and Arbuthnott, Brain Res. 24, 485 (1970); Setler, et al., Eur. J. Pharmacol., 50(4), 419 (1978); and Ungerstedt, et al., "Advances in Dopamine Research" (Kohsaka, Ed.), Pergamon Press, 1982, Oxford, p. 219). 6-OHDA (6-hydroxydopamine) is a neurotoxin used by neurobiologists to selectively kill dopaminergic neurons at the site of injection in the brain in experimental animals. In the 6-OHDA model the nigrostraital dopamine cells are destroyed on one side of the brain (unilateral) by injecting 6-OHDA into the median forebrain bundle, located in front of the substantia nigra. This unilateral injection combined with stimulation by dopamine agonists such as apomorphine will induce rotation behaviour as only one side of the brain is stimulated. Experiments consist of determining a minimum effective dose (MED) to induce rotation for the compound in question. Once a MED has been determined, a second experiment is performed to determine the MED of the compound to overcome Nemonapride block (MED_{Nemonapride}). Nemonapride is a D2 antagonist that blocks the D2 receptor, therefore any observed rotations would be dependent upon activity at the D1 receptor. Finally, once the MED_{Nemonapride} is known a third experiment is run using the MED_{Nemonapride} dose and observing the effect of the D1 antagonist, SCH 23390 alone, the D2 antagonist, Nemonapride alone and finally, the effect of combined treatment with SCH 23390 and Nemonapride. This third experiment confirms the activity of the compound at both receptors as either antagonist alone can only partially inhibit the rotation response induced by the test compound while the combination treatment completely blocks all rotations in the rats (Arnt and Hyttel; Psychophannacology, 85(3), 346 (1985); and Sonsalla, et al., J. Pharmacol Exp. Ther., 247(1), 180, (1988)). This model was validated using apomorphine as the proof-of-principle compound for mixed D1/D2 agonists.

In this model, the active metabolite or Compound **10** and Compound **11** possess 'apomorphine'-like profiles with D1/D2 ratios of about 2 as compared to a ratio of about 3 for apomorphine. Moreover, the duration of action observed was ca. 18h for the compound which is significantly higher than that seen with L-DOPA / apomorphine. A D1 component could not be observed for D2-agonists as exemplified by pramipexole and rotigotine.

### Superiority model

Apomorphine and L-DOPA are able to reverse motility deficits in a mouse model of severe dopamine depletion. Both Apomorphine and L-DOPA stimulate D1 and D2 dopamine receptors. Pramipexole, an agonist at D2-like receptors is ineffective in this model.

The experiments were performed as follows: Mice previously treated with MPTP (2x15mg/kg subcutaneously) and that had stable lesions are used and vehicle treated mice served as normal controls. MPTP (1-methyl-4-phenyl-1,2,3,6-tetrahydropyridine) is a neurotoxin that causes permanent symptoms of Parkinson's disease by killing certain neurons in the substantia nigra of the brain. It is used to study the disease in monkeys and mice. On the day of the experiment, mice were treated with AMPT (250mg/kg subcutaneously) and then returned to their home cages for 1.5 hours after which they are placed in individual cages in the motility unit. AMPT (alpha-methyl-p-tyrosine) is a drug that temporarily reduces brain catecholamine activity (in this case especially dopamine levels). Three hours after the AMPT injection, rescue of locomotive deficits is attempted with The active metabolite or compound 10 and activity was recorded for an additional 1.5 hours. The first 30 min of data collected after the rescue treatment was 'contaminated' due to stressing the animals with handling and injection as evidenced by increased levels in the vehicle controls therefore the data were analyzed using the last 1 hour of recorded data. Various dopaminergic compounds are tested for their ability to reverse the motility deficits produced in this model. Both L-DOPA/Benserazide, and apomorphine restored locomotion in the mice in a dose-dependent manner. Benserazide is a DOPA decarboxylase inhibitor which is unable to cross the blood-brain barrier; it is used to prevent metabolism of L-DOPA to dopamine outside the brain. In contrast, the D2 agonists, pramipexole and bromocriptine did not restore the locomotion in the mice.

This model was used to evaluate whether or not The active metabolite or compound 10 exhibits the same superiority as L-DOPA and apomorphine over D2 agonists. A dose response experiment for was performed and there was a dose-dependent trend for reversing the hypomotility deficits induced by severe depletion of endogenous dopamine. A final experiment directly comparing the effects of apomorphine, pramipexole and compound **10** was performed. It was confirmed that compound **10** was able to restore locomotion in MPTP mice treated and was superior to pramipexole.

### Dyskinesia Rat Model

A rat dyskinesia model reported in the literature (Lundblad, et al., Eur. J Neurosci., 2002, 15, 120) was used to examine the effects of the active metabolite vs. L-DOPA/benserazide with respect to dyskinesias that were assessed as abnormal involuntary movements (AIMs) in 'parkinsonian' rats.

### Study Design

Throughout the study animals received L-DOPA/benserazide (6 mg/kg and 15 mg/kg subcutaneous) or the active metabolite (Compound **10**) (Group B) once daily at t = -20 min. 0 - 180 min. Animals were scored for dyskinesias. Days 1 - 14: All animals were dosed with L-DOPA/benserazide (group A) or the active metabolite (Compound **10**) (Group B).

At days 1, 3, 5, 8 and 12, animals were scored according to AIM-scoring by recording dyskinesias using the Abnormal Involuntary Movement Scale (AIMS) as described previously (Lundblad, et al., Eur. J Neurosci., 2002, 15, 120). Days 15 - 26: Group A animals were treated with the test drug (as group B) instead of L-DOPA/benserazide. Day 15, 16, 17, 19, 22, 24 and 26: Animals scored according AIM-scoring.

### Reversal of L-DOPA-induced dyskinesias in 6-OHDA rats

After eight days of treatment, group A animals had dyskinesia scores of 10-12, which remained constant until day 12. In comparison, group B animals had significantly fewer dyskinesias (scores of 2-4). For group B, the degree of dyskinesias did not change during the study. After shifting group A animals from L-dopa/benserazide to the test drug, their level of dyskinesia gradually decreased to the level observed for the other group of animals. Hence, Compound **11** induced significantly less dyskinesia than L-DOPA and was able to reduce the dyskinesias induced by L-DOPA.

### Anti-Parkinsonian effects in MPTP-treated common marmosets

The experiments were conducted using 6 MPTP treated marmosets (2.0mg/kg daily for up to 5 consecutive days dissolved in sterile 0.9% saline solution). All the animals had previously been treated with L-DOPA (12.5mg/kg p.o., plus carbidopa 12.5mg/kg p.o.) administered daily for up to 30 days in order to induce dyskinesia. Prior to the study all subjects exhibited stable motor deficits including a marked reduction of basal locomotor activity, poor coordination of movement, abnormal and/or rigid posture, reduced alertness and head checking movements. Domperidone was administered 60 min before any of the test compounds. Domperidone is an antidopaminergic drug that suppresses nausea and vomiting. Locomotor Activity was assessed using test cages that are comprised of 8 photo-electric switches comprised of 8 infra-red beams which are strategically placed in the cage and interruption of a beam is recorded as one count. The total number of beam counts per time segment is then plotted as time course or displayed as area under the curve (AUC) for total activity. The assessment of motor disability was performed by a trained observer blinded to the treatment.

L-DOPA (12.5mg/kg, p.o.) increased locomotor activity and reversed motor disability as previously described (Smith, et al. Mov. Disord. 2002, 17(5), 887). The dose chosen for this challenge is at the top of the dose response curve for this drug. Compound **11** (dosed p.o.) as well as compound **10** (dosed subcutaneously) produced dose-related increases in locomotor activity and reversal of motor disability tending to produce in a response greater than for L-DOPA (12.5mg/kg, p.o.). Both test compounds produced a prolonged reversal of motor disability compared to L-DOPA and were as efficacious as L-DOPA.

### In vitro Hepatocyte Assay

Cryopreserved pooled male rat hepatocytes (Sprague Dawley) and pooled human hepatocytes from 10 donors (male and female) were purchased from In Vitro Technologies Inc., BA, USA. Cells were thawed at 37 °C in a water bath, live cells counted and seeded in a total of 100 micro-L in Dulbecco's modified Eagle medium (high glucose) with 5 mM Hepes buffer in 96 well plates, each well containing 250.000 and 500.000 cells/mL for rat and human hepatocytes, respectively. Incubations were started after 15 min of preincubation and stopped at time points of 0, 5, 15, 30 and 60 min for rats and at 0, 30, 60, 90 and 120 min for human hepatocytes. Incubations were stopped by addition of an equal volume of ice-cold acetonitrile containing 10% 1 M HCl. Following centrifugation, 20 micro-L of the supernatants were injected on a HPLC Column Atlantis dC18 3 micro-m, 150 x 2.1 mm i.d. (Waters, MA, USA). The mobile phase had the following composition: A: 5% acetonitrile, 95% H₂0, 3.7 ml/l 25% aq. NH₃, 1.8 mL/L formic acid. Mobile phase B: 100% acetonitrile and 0.1% formic acid. The flow rate was 0.3 ml/min. The gradient operated from 0% to 75 % B from 5 min to 20 min and the eluate was analyzed using a Q-TOFmicro mass spectrometer (Waters, MA, USA). Formation of the product/metabolite was confirmed by accurate mass measurements and comparison with a synthesized standard giving coinciding retention times. In this assay, the metabolism of Compound **11** to Compound **10** was demonstrated.

### Example 4: Pharmacological Testing of Compound 12 (comparative example)

### D₁ cAMP assay

The ability of the compounds to either stimulate or inhibit the D₁ receptor mediated cAMP formation in CHO cells stably expressing the human recombinant D₁ receptor was measured as follows. Cells were seeded in 96-well plates at a concentration of 11000 cells/well 3 days prior to the experiment. On the day of the experiment the cells were washed once in preheated G buffer (1 mM MgCl₂, 0.9 mM CaCl₂, 1 mM IBMX (3-*i*-butyl-1-methylxanthine) in PBS (phosphate buffered saline)) and the assay was initiated by addition of 100 micro-L of a mixture of 30 nM A68930 and test compound diluted in G buffer (antagonism) or test compound diluted in G buffer (agonism).

The cells were incubated for 20 minutes at 37 °C and the reaction was stopped by the addition of 100 micro-L S buffer (0.1 M HCl and 0.1 mM CaCl₂) and the plates were placed at 4 °C for 1h. 68 micro-L N buffer (0.15 M NaOH and 60 mM NaOAc) was added and the plates were shaken for 10 minutes. 60 micro-1 of the reaction were transferred to cAMP FlashPlates (DuPont NEN) containing 40 micro-L 60 mM Sodium acetate pH 6.2 and 100 micro-L IC mix (50 mM Sodium acetate pH 6.2, 0.1 % sodium azide, 12 mM CaCl₂, 1% BSA (bovine serum albumin) and 0.15 micro-Ci/mL ¹²⁵I-cAMP) were added. Following an 18h incubation at 4 °C the plates were washed once and counted in a Wallac TriLux counter. The active metabolite (i.e. Compound **10**) was found to be a D₁ agonist in this assay.

### D₂ cAMP assay

The ability of the compounds to either stimulate or inhibit the D₂ receptor mediated inhibition of cAMP formation in CHO cells transfected with the human D₂ receptor was measure as follows. Cells were seeded in 96 well plates at a concentration of 8000 cells/well 3 days prior to the experiment. On the day of the experiment the cells were washed once in preheated G buffer (1 mM MgCl₂, 0.9 mM CaCl₂, 1 mM IBMX in PBS) and the assay was initiated by addition of 100 micro-1 of a mixture of 1 micro-M quinpirole, 10 microM forskolin and test compound in G buffer (antagonism) or 10 micro-M forskolin and test compound in G buffer (agonism).

The cells were incubated 20 minutes at 37 °C and the reaction was stopped by the addition of 100 micro-1 S buffer (0.1 M HCl and 0.1 mM CaCl₂) and the plates were placed at 4 °C for 1h. 68 micro-L N buffer (0.15 M NaOH and 60 mM Sodium acetate) were added and the plates were shaken for 10 minutes. 60 micro-L of the reaction were transferred to cAMP FlashPlates (DuPont NEN) containing 40 micro-L 60 mM NaOAc pH 6.2 and 100 micro-L IC mix (50 mM NaOAc pH 6.2, 0.1 % Sodium azide, 12 mM CaCl₂, 1% BSA and 0.15 micro-Ci/ml ¹²⁵I-cAMP) were added. Following an 18h incubation at 4 °C the plates were washed once and counted in a Wallac TriLux counter. The active metabolite (i.e. Compound **10**) was found to be a D₂ agonist in this assay.

### D₅ assay

Concentration-dependent stimulation of intracellular Ca²⁺ release by dopamine in hD₅-transfected CHO-Ga16 cells. The cells were loaded with fluoro-4, a calcium indicator dye, for 1h. Calcium response (fluorescence change) was monitored by FLIPR (fluorometric imaging plate reader) for 2.5 min. Peak responses (EC₅₀) were averaged from duplicate wells for each data point and plotted with drug concentrations (cf. Figure 1 for dopamine). The active metabolite (i.e. Compound **10**) was found to be a D₅ agonist in this assay.

### 6-OHDA Rat Model

Dopamine agonists can have activity at either the D1 receptors, the D2 receptors, or both. The rotation response in rats with unilateral 6-OHDA lesions can be used to assess compounds for their ability to stimulate both receptor types and induce rotation (Ungerstedt and Arbuthnott; Brain Res., 24, 485 (1970); Setler, et al., Eur. J. Pharmacol., 50(4), 419 (1978); and Ungerstedt, et al., "Advances in Dopamine Research" (Kohsaka, Ed.), Pergamon Press, 1982, Oxford, p. 219). 6-OHDA (6-hydroxydopamine) is a neurotoxin used by neurobiologists to selectively kill dopaminergic neurons at the site of injection in the brain in experimental animals. In the 6-OHDA model the nigrostraital dopamine cells are destroyed on one side of the brain (unilateral) by injecting 6-OHDA into the median forebrain bundle, located in front of the substantia nigra. This unilateral injection combined with stimulation by dopamine agonists such as apomorphine will induce rotation behaviour as only one side of the brain is stimulated. Experiments consist of determining a minimum effective dose (MED) to induce rotation for the compound in question. Once a MED has been determined, a second experiment is performed to determine the MED of the compound to overcome Nemonapride block (MED_{Nemonapride}). Nemonapride is a D2 antagonist that blocks the D2 receptor, therefore any observed rotations would be dependent upon activity at the D1 receptor. Finally, once the MED_{Nemonapride} is known a third experiment is run using the MED_{Nemonapride} dose and observing the effect of the D1 antagonist, SCH 23390 alone, the D2 antagonist, Nemonapride alone and finally, the effect of combined treatment with SCH 23390 and Nemonapride. This third experiment confirms the activity of the compound at both receptors as either antagonist alone can only partially inhibit the rotation response induced by the test compound while the combination treatment completely blocks all rotations in the rats [Arnt and Hyttel, Psychopharmacology, 1985, 85(3), 346; and Sonsalla et al., J. Pharmacol Exp. Ther., 1988, 247(1), 180]. This model was validated using apomorphine as the proof-of-principle compound for mixed D1/D2 agonists.

In this model, Compounds **10** and **12** possess 'apomorphine'-like profiles with D1 / D2 ratios of about 2-4 as compared to a ratio of about 3 for apomorphine. Moreover, the duration of action observed was ca. 18h for the compound which is significantly higher than that seen with L-DOPA / apomorphine. A D1 component could not be observed for D2-agonists as exemplified by pramipexole and rotigotine.

### Superiority model

Apomorphine and L-DOPA are able to reverse motility deficits in a mouse model of severe dopamine depletion. Both Apomorphine and L-DOPA stimulate D1 and D2 receptors. Pramipexole, an agonist at D2 receptors is ineffective in this model.

The experiments were performed as follows: Mice previously treated with MPTP (2x15mg/kg subcutaneously) and that had stable lesions are used and vehicle treated mice served as normal controls. MPTP (1-methyl-4-phenyl-1,2,3,6-tetrahydropyridine) is a neurotoxin that causes permanent symptoms of Parkinson's disease by killing certain neurons in the substantia nigra of the brain. It is used to study the disease in monkeys and mice. On the day of the experiment, mice were treated with AMPT (250mg/kg subcutaneously) and then returned to their home cages for 1.5 hours after which they were placed in individual cages in the motility unit. AMPT (alpha-methyl-p-tyrosine) is a drug that temporarily reduces brain catecholamine activity (in this case especially dopamine levels). Three hours after the AMPT injection, rescue of locomotive deficits was attempted with Compound **10** and activity was recorded for an additional 1.5 hours. The first 30 min of data collected after the rescue treatment was 'contaminated' due to stressing the animals with handling and injection as evidenced by increased levels in the vehicle controls therefore the data were analyzed using the last 1 hour of recorded data. Various dopaminergic compounds were tested for their ability to reverse the motility deficits produced in this model. Both L-DOPA/Benserazide, and apomorphine restored locomotion in the mice in a dose-dependent manner. Benserazide is a DOPA decarboxylase inhibitor which is unable to cross the blood-brain barrier; it was used to prevent metabolism of L-DOPA to dopamine outside the brain. In contrast, the D2 agonists, pramipexole and bromocriptine did not restore the locomotion in the mice.

This model was used to evaluate whether or not Compound **10** exhibits the same superiority as L-DOPA and apomorphine over D2 agonists. A dose response experiment for was performed and there was a dose-dependent trend for reversing the hypomotility deficits induced by severe depletion of endogenous dopamine. A final experiment directly comparing the effects of apomorphine, pramipexole and Compound **10** was performed. It was confirmed that Compound **10** was able to restore locomotion in MPTP mice treated and was superior to pramipexole.

### Dyskinesia Rat Model

A rat dyskinesia model reported in the literature (Lundblad, et al., Eur. J Neurosci., 2002, 15, 120) was used to examine the effects of Compound **12** vs. L-DOPA/benserazide with respect to dyskinesias that were assessed as abnormal involuntary movements (AIMs) in 'parkinsonian' rats.

### Study Design

Throughout the study animals received L-DOPA/benserazide (6 mg/kg and 15 mg/kg subcutaneous) or Compound **12** (group B) once daily at t = -20 min. 0 - 180 min. Animals were scored for dyskinesias. Days 1 - 14: All animals were dosed with L-DOPA/benserazide (group A) or Compound 12 (group B).

At days 1, 3, 5, 8 and 12, animals were scored according to AIM-scoring by recording dyskinesias using the Abnormal Involuntary Movement Scale (AIMS) as described previously. Days 15 - 26: Group A animals were treated with Compound **12** (as group B) instead of L-DOPA/benserazide. Day 15, 16, 17, 19, 22, 24 and 26: Animals scored according AIM-scoring.

### Results

After eight days of treatment, group A animals had dyskinesia scores of 70-80, which remained constant until day 15. In comparison, group B animals had significantly fewer dyskinesias (scores of 10-25). For group B, the degree of dyskinesias did not change during the study. After shifting group A animals from L-DOPA/benserazide to compound **12** for 10 days, their level of dyskinesia gradually decreased to scores of 30-35. Hence, compound **12** induced significantly less dyskinesia than L-DOPA and was able to reduce the dyskinesias induced by L-DOPA.

### Anti-Parkinsonian effects in MPTP-treated common marmosets

The experiments were conducted using 6 MPTP treated marmosets (2.0mg/kg daily for up to 5 consecutive days dissolved in sterile 0.9% saline solution). All the animals had previously been treated with L-DOPA (12.5mg/kg p.o., plus carbidopa 12.5mg/kg p.o.) administered daily for up to 30 days in order to induce dyskinesia. Prior to the study all subjects exhibited stable motor deficits including a marked reduction of basal locomotor activity, poor coordination of movement, abnormal and/or rigid posture, reduced alertness and head checking movements. Domperidone was administered 60 min before any of the test compounds. Locomotor Activity was assessed using test cages that are comprised of 8 photo-electric switches comprised of 8 infra-red beams which are strategically placed in the cage and interruption of a beam is recorded as one count. The total number of beam counts per time segment is then plotted as time course or displayed as area under the curve (AUC) for total activity. The assessment of motor disability was performed by a trained observer blinded to the treatment.

L-DOPA (12.5mg/kg, p.o.) increased locomotor activity and reversed motor disability as previously described (Smith, et al. Mov. Disord. 2002, 17(5), 887). The dose chosen for this challenge is at the top of the dose response curve for this drug. Compound 12 (dosed p.o.) as well as Compound 10 (dosed p.o.) produced dose-related increases in locomotor activity and reversal of motor disability tending to produce in a response greater than for L-DOPA (12.5mg/kg, p.o.). Both test compounds produced a prolonged reversal of motor disability compared to L-DOPA and were as efficacious as L-DOPA.

## Claims

1. (6aR,10aR)-7-*n*-propyl-6,6a,7,8,9,10,10a,11-octahydro-1,3-dioxa-7-aza-cyclopenta[a]anthracene or a pharmaceutically acceptable salt thereof, for use in reversing dyskinesias, wherein the dyskinesia is associated with dopamine replacement therapy, wherein the dopamine replacement therapy agent is selected from the group consisting of rotigotine, ropinirole, pramipexole, cabergoline, bromocriptine, lisuride, pergolide, L-DOPA and apomorphine.

2. The compound for use according to claim 1 wherein the dyskinesia is established as a result of repeated administration of L-DOPA.

## Patentansprüche

1. (6aR,10aR)-7-*n*-Propyl-6,6a,7,8,9,10,10a,11-octahydro-1,3-dioxa-7-aza-cyclopenta[a]anthracen oder ein pharmazeutisch zulässiges Salz davon, zur Verwendung bei einer Umkehrung von Dyskinesien, wobei die Dyskinesie mit einer Dopaminersatztherapie verbunden ist, wobei der Wirkstoff der Dopaminersatztherapie ausgewählt ist aus der Gruppe, bestehend aus Rotigotin, Ropinirol, Pramipexol, Cabergolin, Bromocriptin, Lisurid, Pergolid, L-DOPA und Apomorphin.

2. Verbindung zur Verwendung nach Anspruch 1, wobei die Dyskinesie aus einer wiederholten Verabreichung von L-DOPA resultiert.

## Revendications

1. (6aR,10aR)-7-*n*-propyl-6,6a,7,8,9,10,10a,11-octahydro-1,3-dioxa-7-aza-cyclopenta[a]anthracène ou un sel pharmaceutiquement acceptable de celui-ci, à utiliser pour inverser une dyskinésie, dans lequel la dyskinésie est associée à une thérapie de remplacement de dopamine, l'agent de thérapie de remplacement de dopamine étant choisi dans le groupe comprenant la rotigotine, le ropinirole, le pramipexole, la cabergoline, la bromocriptine, la lisuride, la pergolide, la L-DOPA et l'apomorphine.

2. Composé à utiliser selon la revendication 1, dans lequel la dyskinésie est établie comme découlant d'une administration répétée de L-DOPA.
